# EUROPEAN PATENT APPLICATION

(11) **EP 4 797 210 A1**
(43) Date of publication of application: **26.08.2026**
(21) Application number: 25171139.6
(22) Date of filing: 17.04.2025
(51) Int. Cl.: G06T 11/00

(54) **METHOD FOR HANDLING DRIFTING GEOMETRIC MISALIGNMENTS IN 3D X-RAY IMAGING**

(30) Priority: 19.02.2025 EP 25158830
(71) Applicant: Siemens Healthineers AG, 91301 Forchheim (DE)
(72) Inventor: Dennerlein, Frank, 90542 Eckental (DE)
(74) Representative: Siemens Healthineers Patent Attorneys

(57) **Abstract**

The invention relates in one aspect to a computer-implemented method for handling drifting geometric misalignments in a projection data set of 3D X-ray imaging for reconstructing a 3D image data set, the method comprising:
- receiving the projection data set, wherein the projection data set comprises a sequence of projection images (I₁, I₂, I₃, ... I_{N}) and a sequence of geometry information (P₁, P₂, P₃, ... P_{N}), wherein each geometry information (P₁, P₂, P₃, ... P_{N}) is associated with the respective projection image (I₁, I₂, I₃, ... I_{N}),
- processing all projection images (I₂, I₃, ... I_{N}) except the first projection image iteratively image-by-image in the order of the sequence, wherein the processing includes
- calculating a cost function C(I_{A},P_{A}) for the current projection image (Iₖ) being processed depending on those projection images (I₁, I₂, I₃, ... Iₖ) and associated geometry information (P₁, P₂, P₃, ... Pₖ) in the sequence up to and including the current projection image (Iₖ), where A = {1, ... k} and k is the index of the current projection image (Iₖ) being processed,
- modifying the geometry information (Pₖ) associated with the current projection image (Iₖ) to numerically minimize the cost function,
- repeating the calculating step and modifying step the current projection image (Iₖ) until a stopping criterium is met,
- reconstructing a 3D image data set on basis of the projection images (I₂, I₃, ... I_{N}) and the associated modified geometry information (P₁, P₂, P₃, ... P_{N}) after the processing step.

## Description

The present disclosure relates to a computer-implemented method for handling drifting geometric misalignments in a projection data set of 3D X-ray imaging for reconstructing an image data set, a data processing unit, a 3D X-ray imaging system, a computer program product and a computer-readable medium.

3D X-ray imaging is about generating a 3D image data set representing the density distribution of an object, in particular a patient, from a collection of a projection data set comprising projection images which are 2D X-ray images and which are acquired from different geometric points of view. This process is called tomographic reconstruction and is generally known.

Over the past decades, several mathematical formulas were derived that achieve such a reconstruction. Those mathematical formulas are the basis of the imaging reconstruction algorithms that are nowadays applied for 3D X-ray imaging. These algorithms generate a 3D image data set from the attenuation signals which are detected by the X-ray detector.

To achieve 3D image data sets of high image quality, it is ideal to know with high precision the geometric information of the points of view from which the projection images were acquired. Advantageously, the contributions of the individual projection images are aligned well to each other during the reconstruction process to generate a high image quality, in particular, sharp, 3D image data set. To the contrary, when the acquisition geometry and therefore the geometric information contains uncertainties, there could be geometric distortions, unsharp edges and/or double contours in the resulting 3D image data set. In this case, the image quality could be as low as the uncertainties render the 3D image data set unuseful.

Geometric uncertainties occur in practice, when a projection image is acquired from a different point of view than what is geometrically assumed. That is, its associated geometric information is not correct. Such a misalignment could be caused by an unprecise motion of the 3D X-ray imaging system and/or by an unknown motion of the object during acquisition.

In general, there are different types of misalignments. Some misalignments might be of global character, i.e., they are constant over the entire acquired sequence of projection images. Other misalignments are of local character, i.e., they differ independently from one projection image to another of the sequence. The latter ones are here called "geometric jitter".

DE 10 2019 216 446 A1 discloses a computer-implemented method for determining the relative geometry of at least two projections acquired from different perspectives by means of transmission imaging of an object through which radiation is transmitted, the at least two projections acquired from different perspectives by means of transmission imaging are provided and the fundamental matrix linking at least two projections is determined at least approximately by solving an optimization problem taking into account a consistency condition existing between the Radon transforms of the at least two projections.

In "A New CT Rawdata Redundancy Measure applied to Automated Misalignment Correction", The 12th International Meeting on Fully Three-Dimensional Image Reconstruction in Radiology and Nuclear Medicine, pages 264 to 267, C. Debbeler et al. publish a new rawdata redundancy measure. In computed tomography, redundantly measured rays (i.e. multiple measurements of a line integral through an object) pose a computationally efficient possibility to quantify the rawdata quality in a cost function and thus to reduce many kinds of artifacts. A general downside of such a cost function is that the portion of redundant rays is generally small and depends on the specific data acquisition geometry. The authors propose using information associated to plane integrals instead of line integrals in order to tremendously increase the rawdata utilization when formulating a cost function that quantifies the rawdata quality. In the cone-beam data acquisition geometry, which does not allow plane integrals to be measured directly, plane-information is obtained using the 2D Radon transform of the measured rawdata and a subsequent differentiation operation. The new rawdata redundancy measure is successfully applied for automatic misalignment correction.

"Suitability of a new alignment correction method for industrial CT", iCT Conference 2014, ISSN 1435-4934, Vol. 19(6), pages 245 to 252, is a paper of Matthias Elter et al. discussing exact knowledge of the geometrical configuration of an industrial CT scanner which is essential for high quality CT image reconstruction. Geometrical misalignment can result in severe misalignment artifacts like blurring and the loss of spatial resolution. In computed tomography, redundantly measured rays (i.e. multiple measurements of a line integral through an object) pose a computationally efficient possibility to quantify the rawdata quality in a cost function and thus to reduce many kinds of artifacts. A general downside of such a cost function is that the portion of redundant rays is generally small and depends on the specific data acquisition geometry. The suitability of using information associated to plane integrals instead of line integrals approach for industrial CT applications is evaluated and proven.

The publication "Epipolar Consistency in Transmission Imaging" of André Aichert et al., IEEE, pages 1 to 15, concerns the optimization of data consistency. This paper presents the derivation of the Epipolar Consistency Conditions (ECC) between two X-ray images from the Beer-Lambert law of X-ray attenuation and the Epipolar Geometry of two pinhole cameras, using Grangeat's theorem. The authors motivate the use of oriented projective geometry to express redundant line integrals in projection images and define a consistency metric, which can be used, for instance, to estimate patient motion directly from a set of X-ray images. The authors describe in detail the mathematical tools to implement an algorithm to compute the Epipolar Consistency metric and investigate its properties with detailed random studies on both artificial and real FD-CT data. A set of 6 reference projections of the CT scan of a fish were used to evaluate accuracy and precision of compensating for random disturbances of the ground truth projection matrix using an optimization of the consistency metric. In addition, the authors use three X-ray images of a pumpkin to prove applicability to real data. The authors conclude, that the metric might have potential in applications related to the estimation of projection geometry. By expression of redundancy between two arbitrary projection views, the authors in fact support any device or acquisition trajectory which uses a cone-beam geometry. The authors discuss certain geometric situations, where the ECC provide the ability to correct 3D motion, without the need for 3D reconstruction.

However, there is also another type of misalignment, neither purely global nor purely local. It is a misalignment that undergoes small changes from one projection images to another of the sequence, incrementally increasing or decreasing in one or some geometric parameters. Examples of this type of misalignment are drifts in the X-ray focal spot, drifts in an object location or orientation, or continuous motion patterns of a scanned patient. These misalignments are called "drifting geometric misalignments" and can accumulate incrementally. Up to now, the way of coping with a projection data set that includes drifting geometric misalignments is to directly apply conventional jitter reduction on this projection data set.

The inventor is aware of the following conventional jitter reduction method:
- receiving the projection data set, wherein the projection data set comprises a sequence of projection images (I₁, I₂, I₃, ... I_{N}) and a sequence of geometry information (P₁, P₂, P₃, ... P_{N}), wherein each geometry information (P₁, P₂, P₃, ... P_{N}) is associated with the respective projection image (I₁, I₂, I₃, ... I_{N}),
- processing all projection images (I₁, I₂, I₃, ... I_{N}) iteratively image-by-image in an arbitrary order of the sequence, wherein the processing includes
- calculating a cost function C(I_{A},P_{A}) for the current projection image (Iₖ) being processed depending on all projection images (I₁, I₂, I₃, ... I_{N}) and associated geometry information (P₁, P₂, P₃, ... P_{N}), where k is the index of the current projection image (Iₖ) being processed,
- modifying the geometry information (Pₖ) associated with the current projection image (Iₖ) to numerically minimize the cost function,
- repeating the calculating step and modifying step the current projection image (Iₖ) until a stopping criterium is met,
- reconstructing a 3D image data set on basis of the projection images (I₁, I₂, I₃, ... I_{N}) and the associated modified geometry information (P₁, P₂, P₃, ... P_{N}) after the processing step.

This approach produces regularly good outcomes in many scenarios. However, there are cases of drifting geometric misalignments when such conventional jitter reduction method completely fails. In these cases, no misalignment might be detected at all, even though notable misalignment patterns do exist. Therefore, jitter reduction yields insufficient 3D image data sets in such cases. One reason is that the degrees of freedom in misalignment estimation in above conventional jitter reduction method are too large causing not a stable and robust performance in cases of drifting geometric misalignments.

From other approaches it is known that it is possible to implement compensation methods that explicitly incorporate motion models of drifting geometric misalignments. This is, for example, done in cardiac computed tomography (CT) or C-arm CT, where periodic (drifting) motions are present. Also, for Dental cone beam CT or orthopedic 3D imaging, it is typical to involve such motion models that are depending on human joints and/or motion ranges. Apparently, such motion models are application specific. It is difficult to develop a well-generalized motion model that performs well in some or even all relevant types of applications, such as in medical as well as industrial 3D X-ray imaging. Furthermore, using an explicit motion model can result in a significant change in the misalignment compensation algorithm, which is not wanted by the inventor.

The underlying technical problem of the invention is to provide a better computer-implemented method for handling drifting geometric misalignments in a projection data set of 3D X-ray imaging for reconstructing a 3D image data set, a better data processing unit, a better 3D X-ray imaging system, a better computer program product and a better computer-readable medium wherein in particular the image quality of the reconstructed 3D image data set is improved.

This problem is solved by the features of the independent claims. Advantageous embodiments are disclosed within the dependent claims.

The invention relates in one aspect to a computer-implemented method for handling drifting geometric misalignments in a projection data set of 3D X-ray imaging for reconstructing a 3D image data set, the method comprising:
- receiving the projection data set, wherein the projection data set comprises a sequence of projection images (I₁, I₂, I₃, ... I_{N}) and a sequence of geometry information (P₁, P₂, P₃, ... P_{N}), wherein each geometry information (P₁, P₂, P₃, ... P_{N}) is associated with the respective projection image (I₁, I₂, I₃, ... I_{N}),
- processing all projection images (I₂, I₃, ... I_{N}) except the first projection image iteratively image-by-image in the order of the sequence, wherein the processing includes
- calculating a cost function C(I_{A},P_{A}) for the current projection image (Iₖ) being processed depending on those projection images (I₁, I₂, I₃, ... Iₖ) and associated geometry information (P₁, P₂, P₃, ... Pₖ) in the sequence up to and including the current projection image (Iₖ), where A = {1, ... k} and k is the index of the current projection image (Iₖ) being processed,
- modifying the geometry information (Pₖ) associated with the current projection image (Iₖ) to numerically minimize the cost function,
- repeating the calculating step and modifying step the current projection image (Iₖ) until a stopping criterium is met,
- reconstructing a 3D image data set on basis of the projection images (I₁, I₂, I₃, ... I_{N}) and the associated modified geometry information (P₁, P₂, P₃, ... P_{N}) after the processing step.

The invention relates in one aspect to a data processing unit, comprising a processor and a memory comprising instructions stored thereon which, when executed by the processor, cause the data processing unit to perform the method according to the invention.

The invention relates in one aspect to a 3D X-ray imaging system, comprising the data processing unit according to the invention, an X-ray source for emitting X-rays, an X-ray detector for acquiring X-rays having passed an object, wherein the data processing unit is configured to receive the projection data set which is based on the detected X-rays. The 3D X-ray imaging system can be configured for cone-beam computed tomography. The 3D X-ray imaging system is in particular configured for medical imaging and/or industrial imaging applications. The 3D X-ray imaging system can be a medical computed tomography (CT) imaging system, a C-arm CT imaging system, a cardiac CT imaging system, a dental CT imaging system, an orthopedic CT imaging system, an industrial CT imaging system, a nondestructive testing CT imaging system, and/or a customs inspections CT imaging system.

The X-ray source may comprise a thermionic emitter or a cold emitter. There could be also several X-ray sources. The X-ray source or the X-ray sources could be mounted movable around the object or spatially fixed to the patient. The X-rays detector could be mounted movable around the object or could be arranged spatially fixed to the patient.

The invention relates in one aspect to a computer program product, comprising a computer program, the computer program being loadable into a memory unit of a data processing unit, including program code sections to make the data processing unit execute the method according to the invention when the computer program is executed in said data processing unit.

The invention relates in one aspect to a computer-readable medium, on which program code sections of a computer program are saved, said program code sections being loadable into and/or executable in a data processing unit to make the data processing unit execute the method according to the invention when the program code sections are executed in said data processing unit.

The advantages of the invention are as follows:
The invention yields a beneficial modification of a well-established, theoretically sound and practically successful, conventional jitter reduction method. The invention advances the conventional method into being capable of robustly estimating and compensating for drifting geometric misalignments.

One difference between the method according to the invention and the conventional method is in the calculation of the cost function that is according to the invention optimized in order to improve geometry information for a given 3D X-ray projection data set. In the conventional method, the cost function is defined to use all data as input, and therefore it measures the consistency of the entire projection data set. That is, the cost function of the known solution involves several projection images which have not yet been processed during the cost function minimization process. The equation of the cost function is independent of the index of the current projection image that is currently optimized. In other words, this equation is constant during the entire processing step.

In the method according to the invention, the equation of the cost function varies during the processing step image-by-image: it (only) involves data with a lower or equal projection index with reference to the current projection image that is currently optimized. The cost function of the proposed solution depends in particular on data which has been already optimized. The geometry information of the currently processed projection image is therefore made consistent with the previous projection images, disregarding any data that might come after the current projection image in indices bigger than k. The method according to the invention is in particular handling drifting geometric misalignment incrementally. This makes the optimization beneficially more robust. In evaluations based on simulated data or real data, the method according to the invention clearly outperforms the conventional method, in particular, in scenarios of drifting geometric misalignments.

Another advantage is that the method according to the invention requires minor implementation changes over the conventional method. In particular, the need to apply explicit major changes to the cost function (such as additional terms that enforce slowly changing parameters in the motion patterns) does not exist. Complexity that would come with these explicit changes is advantageously avoided.

The computer-implemented method according to the invention is in particular executable via the data processing unit. The computer-implemented method is in particular configured for reconstructing the 3D image data set.

Handling drifting geometric misalignments means in particular reducing drifting geometric misalignments and/or optimizing image quality. The drifting geometric misalignments can comprise at least one of drifts in X-ray focal spot position, in object location of the object of the projection data set, in object orientation of the object, or in continuous motion patterns of the object. Drifting geometric misalignments occur in particular over time and/or during the sequence.

The projection data set of 3D X-ray imaging is in particular a projection data set acquired via a 3D X-ray imaging system. The projection data set comprises in particular projection images of the object allowing a reconstruction of the 3D image data set of the object. The object can in particular be a patient or a device or a material. 3D X-ray imaging can be medical imaging and/or industrial imaging.

The projection data set comprises in particular a plurality of projection images and a plurality of associated geometry information. The projection data set comprises in particular a number of at least three projection images and an equal number of associated geometry information. The number can be higher than three, for example, more or equal than 20, or preferably more or equal than 100. The number is typically as high that the 3D image data set is reconstructed on basis of projection images acquired over scan projection angles covering at least 180° of the object.

The projection images are typically 2D X-ray images. A projection image comprises in particular an attenuation profile or attenuation contour resulting from absorption and/or attenuation of emitted X-rays within the object. The attenuation profile or attenuation contour reflects in particular the density distribution of the object. A projection image is typically based on the X-rays detected by the X-ray detector. It is possible that the X-ray detector is configured to generate a projection image on basis on the detected X-rays. Additionally or alternatively, the data processing unit can be configured to generate a projection image on basis on the detected X-rays. The detected X-rays are typically converted into X-ray signals prior to their conversion into a projection image. The projection images can be buffered and/or stored in a memory, e.g. of the 3D X-ray imaging system or the data processing unit or a network-based storage system,

The projection images are typically acquired subsequently and therefore typically vary in their projection acquisition time. It is an option that some or all projection images are typically acquired at the same time. The projection acquisition time of a projection image is defined with reference to the time when the emitted X-rays pass the object and/or are detected by the X-ray detector.

The projection images are typically acquired from different scan projection angles and optionally at different longitudinal positions of the object. The difference between respective scan projection angels is ideally constant. Alternatively, the differences between at least three projection images may vary due to the different type of misalignments.

The geometry information of the respective projection images varies typically at least due to the different respective scan projection angle. The geometry information defines mathematically and/or geometrically the position from where the X-rays represented in the individual projection image are emitted and/or where those X-rays are detected. Associated means, that by definition a projection image and the respective geometry information are linked. Resorting the projection images doesn't break up their associations with the respective geometry information. That is, by resorting the projection images automatically the associated geometry information is also resorted and vice versa.

Typically, each geometry information can be either determined prior to, during, or after acquiring the respective projection images. The determining can comprise sensing, measuring, recognizing, and/or calculating the geometry information. Additionally or alternatively, the geometry information can be buffered and/or stored in the memory and/or loaded from such memory.

The projection data set is in particular received at an interface of the data processing unit. The interface can be wired or wireless. The projection data set can be transferred, for example, from the memory via a network connecting the memory or the 3D X-ray imaging system with the data processing unit and/or via other data connections.

The sequence of projection images is typically in the same sequence as the geometry information. That is, at the processing of the current projection image in particular the associated geometry information and not any other arbitrary geometry information is used.

The processing occurs typically via the processor of the data processing unit. The processor can be of any type of regular processor as known in the art, including physical processors and/or virtualized processors and/or cloud-based processors and/or a combination thereof.

The processing step loops over all projection images except the first projection image. Having N projection images, the processing step is executed at least N-1 times. The processing step is executed iteratively, that is, repeated for the current projection image until the stopping criterium is met. The calculating step and modifying step are executed at least N-1 times, regularly much more often, but not with the first projection image as current projection image.

The processing step is executed image-by-image in a series. That is, not two projection images are processed in parallel.

The processing step is executed in the order of the sequence. That is, the projection images as ordered in the sequence at the beginning are processed one-by-another without a random selection of the upcoming current projection image.

For example, the cost function of the second projection image depends on the first projection image and its associated geometry information, and the second projection image and its associated geometry information. The cost function of the third projection image depends on the first projection image and its associated geometry information, the second projection image and its associated geometry information, and the third projection image and its associated geometry information.

The processing step includes for each projecting image being processed at least two steps: the modifying step and the calculating step. Each of both steps can comprise one or several partial steps to be executed.

The calculating the cost function occurs generally as known in the art, however, including the input values according to the invention. The cost function in particular quantifies inconsistency between those projection images and associated geometry information in the sequence up to and including the current projection image. The cost function is in particular computed from values of the projection images and/or can be based on measuring deviations of redundant data in the projection domain. The lower the value of the cost function is, the better is the data consistency and the lower is the inconsistency. Good consistency means that the geometry information advantageously describes accurately the points of view from where the projection images have been acquired.

The modifying the geometry information associated with the current projection image to numerically minimize the cost function occurs generally as known in the art. Minimizing the cost functions means in particular optimizing the cost function. The modifying the geometry information associated with the current projection image occurs for instance based on some practical numerical optimization routines, until an updated geometry information for the current projection image is found that leads towards a minimization of the cost function. The modified geometry information replaces the previous geometry information associated with the current projection image.

Repeating the calculating step and modifying step means that instead of the previous geometry information associated with the current projection image before the modifying, for the next minimization cost function calculation the modified geometry information associated with the current projection image and the remaining previous input values are used for calculating the cost function.

One stopping criterium is that the cost function value is below a certain threshold. Another stopping criterium is that a counter of the repetition reaches a specific limit or that a time limit for the processing of the current projection image would be exceeded unless the repetition is stopped. The stopping criterium function is typically evaluated and/or executed prior to starting a new round of repeating the calculating and modifying step.

The modifying the geometry information may comprise modifying a parameter of the geometry information pre-selected by a user, wherein the parameter is selected and received prior to the processing. In particular the user of the 3D X-ray imaging system and/or the data processing unit may select the parameter via an input device of the 3D X-ray imaging system and/or an input device of the data processing unit. One or more parameters may be pre-selected by the user. It is possible that the modifying the geometry information is restricted to modifying only one or more parameters pre-selected by the user such that other parameters which are not pre-selected cannot be modified. The modifying the parameter comprises particular an updating of the parameter.

The modifying the geometry information may comprise modifying the value of the parameter within a limit pre-defined by the user, wherein the limit is defined and received prior to the processing. In particular the user of the 3D X-ray imaging system and/or the data processing unit may define the limit via an input device of the 3D X-ray imaging system and/or an input device of the data processing unit. One or more limits may be pre-defined by the user. It is possible, that the modifying the geometry information is restricted to modifying a value of one or more parameters only within the limit pre-defined by the user. The modifying a value of the parameter comprises particular an updating of the value within the limit of the parameter.

Ideally, the input device and/or the 3D X-ray imaging system are configured to transfer the pre-selected parameter and/or the pre-defined limit to the data processing unit prior to the processing step. The user-definition and user-selection are in particular beneficial if only those parameters are selected and/or those limits are defined which are the ones where a misalignment is likely to occur.

After N-1 projection images have been processed typically the 3D image data set is reconstructed. The reconstructing the 3D image data set on basis of the projection images and the associated modified geometry information generally occurs as known in the art. The reconstructed 3D image data set may be provided via a display device and/or stored in the memory. Advantageously, the reconstructed 3D image data set comprises a high quality, preferably sharp, representation of the object.

One embodiment of the invention relates in one aspect to that the sequence of projection images and the sequence of geometry information are resorted prior to the processing. The resorting may occur by the scan projection angle or the projection acquisition time or an ECG information or a temperature information.

The data processing unit can be a computer device or a computer network device or a cloud computing device or a handheld device. The data processing unit can be realized as a data processing system or as a part of a data processing system. The data processing system can, for example, comprise cloud-computing system, a computer network, a computer, a tablet computer, a smartphone or the like. The data processing system can comprise hardware and/or software. The hardware can be, for example, a processor system, a memory system and combinations thereof. The hardware can be configurable by the software and/or be operable by the software.

The computer program product can be, for example, a computer program or comprise another element apart from the computer program. This other element can be hardware, for example a memory device, on which the computer program is stored, a hardware key for using the computer program and the like, and/or software, for example a documentation or a software key for using the computer program.

Reference is made to the fact that the described methods and the described data processing unit as well as the described device are merely preferred example embodiments of the invention and that the invention can be varied by a person skilled in the art, without departing from the scope of the invention provided it is specified by the claims. Said features, advantages or alternative embodiments of the apparatus apply also to the method and vice-versa.

Independent of the grammatical term usage, individuals with male, female or other gender identities are included within the term.

The invention will be illustrated below with reference to the accompanying figures using example embodiments. The illustration in the figures is schematic and highly simplified and not necessarily to scale.
Fig. 1 shows a conventional jitter reduction method,
Fig. 2 shows a method according to the invention,
Fig. 3 shows the method according to the invention in another figure and
Fig. 4 shows a first exemplary embodiment of the method.

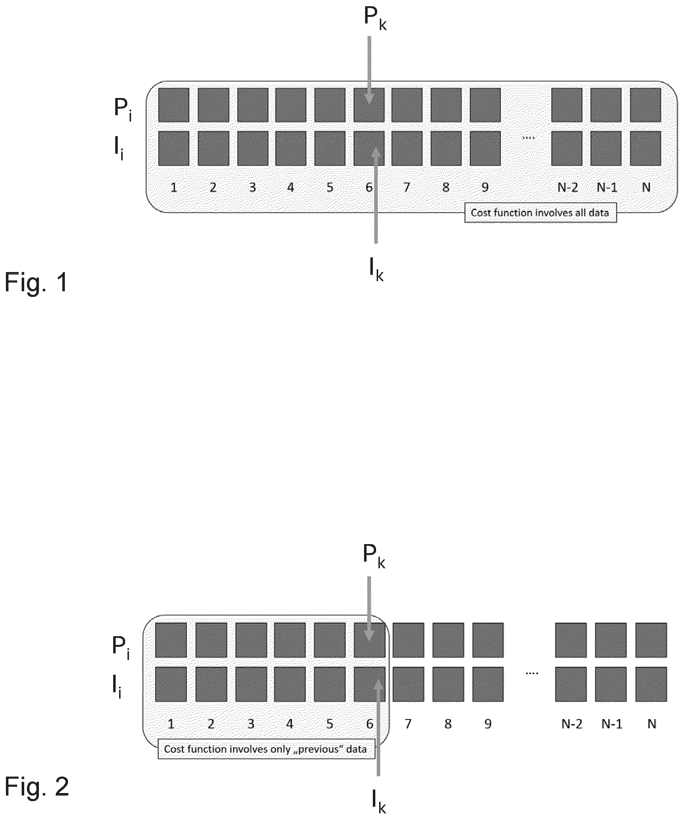

**Fig. 1** shows a schematic view of a conventional jitter reduction method.

The conventional jitter reduction method comprises the steps:
- receiving the projection data set, wherein the projection data set comprises a sequence of projection images I₁, I₂, I₃, ... I_{N} and a sequence of geometry information P₁, P₂, P₃, ... P_{N}, wherein each geometry information P₁, P₂, P₃, ... P_{N} is associated with the respective projection image I₁, I₂, I₃, ... I_{N},
- processing all projection images I₁, I₂, I₃, ... I_{N} iteratively image-by-image in an arbitrary order of the sequence, wherein the processing includes
- calculating a cost function C(I_{A},P_{A}) for the current projection image Iₖ being processed depending on all projection images I₁, I₂, I₃, ... I_{N} and associated geometry information P₁, P₂, P₃, ... P_{N}, where k is the index of the current projection image Iₖ being processed,
- modifying the geometry information Pₖ associated with the current projection image Iₖ to numerically minimize the cost function,
- repeating the calculating step and modifying step the current projection image Iₖ until a stopping criterium is met,
- reconstructing a 3D image data set on basis of the projection images I₁, I₂, I₃, ... I_{N} and the associated modified geometry information P₁, P₂, P₃, ... P_{N} after the processing step.

**Fig. 2** shows a schematic view of a method according to the invention.

The computer-implemented method for handling drifting geometric misalignments in a projection data set of 3D X-ray imaging for reconstructing a 3D image data set, the method comprises the steps:
- receiving the projection data set, wherein the projection data set comprises a sequence of projection images I₁, I₂, I₃, ... I_{N} and a sequence of geometry information P₁, P₂, P₃, ... P_{N}, wherein each geometry information P₁, P₂, P₃, ... P_{N} is associated with the respective projection image I₁, I₂, I₃, ... I_{N},
- processing all projection images I₂, I₃, ... I_{N} except the first projection image iteratively image-by-image in the order of the sequence, wherein the processing includes
- calculating a cost function C(I_{A},P_{A}) for the current projection image Iₖ being processed depending on those projection images I₁, I₂, I₃, ... Iₖ and associated geometry information P₁, P₂, P₃, ... Pₖ in the sequence up to and including the current projection image Iₖ, where A = {1, ... k} and k is the index of the current projection image Iₖ being processed,
- modifying the geometry information Pₖ associated with the current projection image Iₖ to numerically minimize the cost function,
- repeating the calculating step and modifying step the current projection image Iₖ until a stopping criterium is met,
- reconstructing a 3D image data set on basis of the projection images I₁, I₂, I₃, ... I_{N} and the associated modified geometry information P₁, P₂, P₃, ... P_{N} after the processing step

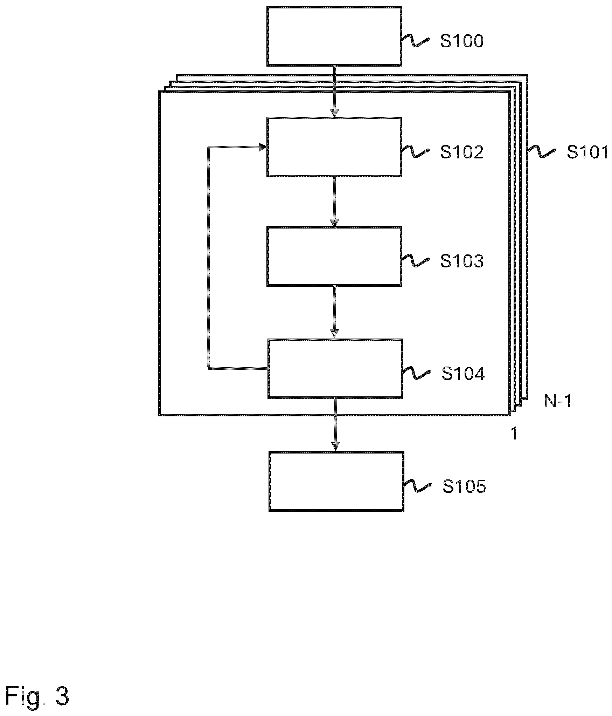

**Fig. 3** shows a flow diagram of the method according to the invention.

Step S100 denotes receiving the projection data set, wherein the projection data set comprises a sequence of projection images I₁, I₂, I₃, ... I_{N} and a sequence of geometry information P₁, P₂, P₃, ... P_{N}, wherein each geometry information P₁, P₂, P₃, ... P_{N} is associated with the respective projection image I₁, I₂, I₃, ... I_{N}.

Step S101 denotes processing all projection images I₂, I₃, ... I_{N} except the first projection image iteratively image-by-image in the order of the sequence. That is, step S101 is executed N-1 times.

Step S102 denotes that the processing includes calculating a cost function C(I_{A},P_{A}) for the current projection image Iₖ being processed depending on those projection images I₁, I₂, I₃, ... Iₖ and associated geometry information P₁, P₂, P₃, ... Pₖ in the sequence up to and including the current projection image Iₖ, where A = {1, ... k} and k is the index of the current projection image Iₖ being processed. The cost function C(I_{A},P_{A}) quantifies inconsistency between those projection images I₁, I₂, I₃, ... Iₖ and associated geometry information P₁, P₂, P₃, ... Pₖ in the sequence up to and including the current projection image. The cost function C(I_{A},P_{A}) is based on measuring deviations of redundant data in the projection domain.

Step S103 denotes that the processing further includes modifying the geometry information Pₖ associated with the current projection image Iₖ to numerically minimize the cost function.

Step S104 denotes that the processing further includes repeating the calculating step and modifying step the current projection image Iₖ until a stopping criterium is met.

Step S105 denotes reconstructing a 3D image data set on basis of the projection images I₁, I₂, I₃, ... I_{N} and the associated modified geometry information P₁, P₂, P₃, ... P_{N} after the processing step.

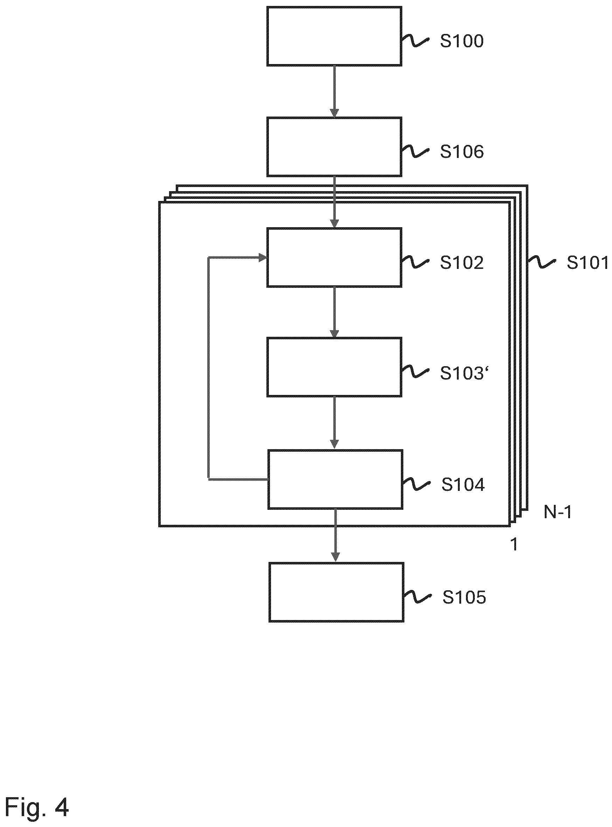

**Fig. 4** shows a flow diagram of the first exemplary embodiment of the method.

Step S106 denotes that the sequence of projection images I₁, I₂, I₃, ... I_{N} and the sequence of geometry information P₁, P₂, P₃, ... P_{N} are resorted prior to the processing. The resorting may occur by the scan projection angle or the projection acquisition time or by an ECG information or a temperature information.

Step S103' denotes that the processing further includes modifying the geometry information Pₖ associated with the current projection image Iₖ to numerically minimize the cost function. The modifying the geometry information P₁, P₂, P₃, ... Pₖ comprises modifying a parameter of the geometry information P₁, P₂, P₃, ... Pₖ pre-selected by a user, wherein the parameter is selected and received prior to the processing. The modifying the geometry information P₁, P₂, P₃, ... Pₖ comprises modifying the value of the parameter within a limit pre-defined by the user, wherein the limit is defined and received prior to the processing.

Although the invention has been illustrated and described in detail by the preferred embodiments, the invention is not limited to the disclosed examples and other variations can be derived by a person skilled in the art without departing from the scope of the invention.

## Claims

1. A computer-implemented method for handling drifting geometric misalignments in a projection data set of 3D X-ray imaging for reconstructing a 3D image data set, the method comprising:
- receiving the projection data set, wherein the projection data set comprises a sequence of projection images (I1, I2, I3, ... IN) and a sequence of geometry information (P1, P2, P3, ... PN), wherein each geometry information (P1, P2, P3, ... PN) is associated with the respective projection image (I1, I2, I3, ... IN),
- processing all projection images (I2, I3, ... IN) except the first projection image iteratively image-by-image in the order of the sequence, wherein the processing includes
- calculating a cost function C(IA,PA) for the current projection image (Ik) being processed depending on those projection images (I1, I2, I3, ... Ik) and associated geometry information (P1, P2, P3, ... Pk) in the sequence up to and including the current projection image (Ik), where A = {1, ... k} and k is the index of the current projection image (Ik) being processed,
- modifying the geometry information (Pk) associated with the current projection image (Ik) to numerically minimize the cost function,
- repeating the calculating step and modifying step the current projection image (Ik) until a stopping criterium is met,
- reconstructing a 3D image data set on basis of the projection images (I1, I2, I3, ... IN) and the associated modified geometry information (P1, P2, P3, ... PN) after the processing step.

2. The method as claimed in claim 1,
wherein the sequence of projection images (I1, I2, I3, ... IN) and the sequence of geometry information (P1, P2, P3, ... PN) are resorted prior to the processing.

3. The method as claimed in claim 2,
wherein the resorting occurs by the scan projection angle or the projection acquisition time.

4. The method as claimed in claim 2,
wherein the resorting occurs by an ECG information or a temperature information.

5. The method as claimed in any preceding claim,
wherein modifying the geometry information (P1, P2, P3, ... Pk) comprises modifying a parameter of the geometry information (P1, P2, P3, ... Pk) pre-selected by a user, wherein the parameter is selected and received prior to the processing.

6. The method as claimed in claim 5,
wherein modifying the geometry information (P1, P2, P3, ... Pk) comprises modifying the value of the parameter within a limit pre-defined by the user, wherein the limit is defined and received prior to the processing.

7. The method as claimed in any preceding claim,
wherein the cost function C(IA,PA) quantifies inconsistency between those projection images (I1, I2, I3, ... Ik) and associated geometry information (P1, P2, P3, ... Pk) in the sequence up to and including the current projection image (Ik).

8. The method as claimed in any preceding claim,
wherein the cost function C(IA,PA) is based on measuring deviations of redundant data in the projection domain.

9. The method as claimed in any preceding claim,
wherein the drifting geometric misalignments comprise at least one of drifts in X-ray focal spot position, in object location of the object of the projection data set, in object orientation of the object, or in continuous motion patterns of the object.

10. Data processing unit, comprising
a processor, and
a memory comprising instructions stored thereon which, when executed by the processor, cause the data processing unit to perform the method of any of claims 1 to 9.

11. 3D X-ray imaging system, comprising
- the data processing unit of claim 10,
- an X-ray source for emitting X-rays,
- an X-ray detector for acquiring X-rays having passed an object,
- wherein the data processing unit is configured to receive the projection data set which is based on the detected X-rays.

12. A computer program product, comprising a computer program, the computer program being loadable into a memory of a data processing unit, including program code sections to make the data processing unit execute the method of any one of claims 1 to 9 when the computer program is executed in said data processing unit.

13. A computer-readable medium, on which program code sections of a computer program are saved, said program code sections being loadable into and/or executable in a data processing unit to make the data processing unit execute the method of any one of the claims 1 to 9 when the program code sections are executed in said data processing unit.
